Europäisches Patentamt

European Patent Office

Office européen des brevets

(19)

(11) Publication number: **0 417 819 A1**

# EUROPEAN PATENT APPLICATION

(21) Application number: 90201952.0

(51) Int. Cl.5: **C07K 7/08, A61K 37/02**

(22) Date of filing: **18.07.90**

(30) Priority: **11.08.89 NL 8902053**

(43) Date of publication of application:
**20.03.91 Bulletin 91/12**

(84) Designated Contracting States:
**AT BE CH DE DK ES FR GB GR IT LI NL SE**

(71) Applicant: AKZO N.V.
Velperweg 76
NL-6824 BM Arnhem(NL)

(72) Inventor: **Van Nispen, Johannes Wilhelmus Franciscus Maria**
**Beethovengaarde 131**
**NL-5344 CE Oss(NL)**

(74) Representative: **Hermans, Franciscus G.M. et al**
**Patent Department AKZO N.V. Pharma**
**Division P.O. Box 20**
**NL-5340 BH Oss(NL)**

(54) Psycho-pharmacological peptides.

(57) Disclosed are novel (6-17) $\beta$-endorphin fragments which possess a derivatized lysine at site 9.
The peptides have the amino acid sequence according to the general formula:

$$\underset{\text{(I)}}{} \quad \overset{6}{\text{Thr}}-\overset{7}{\text{R}_1}-\overset{8}{\text{L-Glu}}-\overset{9}{\text{X}}-\overset{10}{\text{L-Ser}}-\overset{11}{\text{R}_2}-\overset{12}{\text{L-Thr}}-\overset{13}{\text{Pro}}-\overset{14}{\text{L-Leu}}-\overset{15}{\text{Val}}-\overset{16}{\text{Thr}}-\overset{17}{\text{R}_3}$$

wherein X is a derivatized lysine, selected from the group Lys-Ac, Lys(Z), des-Lys, $A_2$hy, Nle, Met, Leu or Lys [-(ar)alkyl]; $R_1$ is Ser, Ala or Pro; $R_2$ is L-Gln, D-Gln, Glu(tyramine) or iodine-containing derivatives thereof; and $R_3$ is L-Leu, Leu-NHCH₃, Met, Phe, Phe(Cl), Phe(I), Cha, Mag, Val, (HO)Leu, Bua or Bug.
Methods of making the fragments are also described.

EP 0 417 819 A1

## PSYCHO-PHARMACOLOGICAL PEPTIDES

### Background of the invention

#### Field

The present invention relates to peptides derived from a fragment of $\beta$-lipotropic hormone ($\beta$-LPH).

#### State of the Art.

Human B-lipotropic hormone consists of 89 amino acids and has fat-mobilizing activity.

Fragments of this hormone having different functions have already been described in the literature. Fragments (39-56) of $\beta$-LPH have, for example, an effect on the melanocytes and consequently influences the pigmentation of the skin. $\beta$-endorphins,(59-89) fragments of $\beta$-LPH, have an analgesic effect to which naloxone has an antagonistic action. The analgesic effect of morphine can likewise be antagonized by naloxone, so that the conclusion that both morphine and $\beta$-endorphins have an influence on one and the same receptor is justified.

Furthermore it has already been found that $\beta$-endorphin possesses certain psychopharmacological properties. Thus, this peptide delays the extinction of the (active) avoidance behaviour of rats in the well-known pole climbing test (pole jumping avoidance behaviour). This characteristic of $\beta$-endorphin cannot be antagonized by known morphine antagonists such as naloxone or natrexone, so that the conclusion that the psychopharmacological action of $\beta$-endorphin is established independently of the opiate receptor sites in the brain is certainly justified.

In addition to $\beta$-endorphin, the smaller peptide fragments $\alpha$-endorphin and met-enkephalin derived therefrom were also found to inhibit the extinction of the avoidance behaviour in a similar way.

The peptide $\gamma$-endorphin, $\beta$-endorphin (1-17), which differs from $\alpha$-endorphin by the presence of just one additional amino acid (leucine) at the C-terminal side, was also found to have a psychopharmacological action, albeit of a totally different nature to that of $\alpha$- and $\beta$-endorphin. Whereas with $\alpha$-endorphin the extinction of the avoidance behaviour was retarded, $\gamma$-endorphin, in contrast, was found to accelerate the extinction of the avoidance behaviour. It is striking that the addition of one amino acid residue at the C-terminal part of $\alpha$-endorphin causes such a dramatic reversal of the effect on the behaviour. Apart from $\gamma$-endorphin itself, derivatives of $\gamma$-endorphin have also been published, see German Patent Application 2,826,533 corresponding to GB 2000151. This patent application describes, inter alia , the $\gamma$-endorphin derivative $\beta$-endorphin having with analgesic, antidepressive, calming, sedative and hypnotic properties.

In European Patent Application EP-A-0,004,394 (corresponding to U.S. Patent No. 4,256,736) it is stated that peptides with the amino acid sequence of $\beta$-endorphin (2-17), or closely related analogues derived therefrom, accelerate the extinction of the avoidance behaviour to a greater extent than is the case with $\gamma$-endorphin. In addition, in contrast to $\gamma$-endorphin, these peptides have no further affinity whatsoever for the opiate receptors.

It has also been found that the complete sequence 2-17 of $\beta$-endorphin is not necessary to maintain the accelerated extinction activity (European Patent No. 0,015,036 corresponding to U.S. Patent no. 4,271,152).

However, the peptides from the two last-mentioned references have disadvantage. They are not metabolically stable. Consequently, side effects can arise as a result of the peptide fragments formed during metabolic degradation.

The synthesis of several peptides related to $\beta$-LPH (62-77) but shortened from the N-terminal end is described in Greven, H.M. et al, "Synthesis of fragments of human $\beta$-lipotropin, B$_h$-LPH Part IV. The synthesis of shortened peptides related to des-1-tyrosine-$\gamma$-endorphin [$\beta$-LPH-(62-77)]", Journal of the Royal Netherlands Chemical Society , 99/9, pp. 284-286 (Sept. 1980). In the described syntheses, use was made of the classical fragment condensation approach.

### Summary of the Invention

The invention includes a novel (6-17) β-endorphin fragment which possesses a derivatized lysine at site 9. These endorphin fragments are surprisingly metabolically stable.

The novel peptides according to the invention contain at least the amino acid sequence according to the general formula I:

$$6 \quad 7 \quad 8 \quad 9 \quad 10 \quad 11 \quad 12 \quad 13 \quad 14 \quad 15 \quad 16 \quad 17$$

$$(I) \quad Thr\text{-}R_1\text{-}L\text{-}Glu\text{-}X\text{-}L\text{-}Ser\text{-}R_2\text{-}L\text{-}Thr\text{-}Pro\text{-}L\text{-}Leu\text{-}Val\text{-}Thr\text{-}R_3$$

wherein X is a derivatized lysine, selected from the group Lys(Ac), Lys(Z), des-Lys, $A_2$hy, Nle, Met, Leu and Lys [(ar)alkyl],

$R_1$ is Ser, Ala or Pro,

$R_2$ is L-Gln, D-Gln, Glu(tyramine), or iodine-containing derivatives thereof, and

$R_3$ is L-Leu, Leu-NHCH$_3$, Met, Phe, Phe(Cl), Phe(I), Cha, Mag, Val, (HO)Leu, Bua or Bug.

As already described in European Patent Specification No. 0,015,036, the 4 N-terminal amino acids of sequence 2-17 are not essential for the action of the γ-endorphin. It is, however, very well possible for one or more of these N-terminal amino acids to be present in the peptides according to the invention without the activity being impaired as a result. The amino acid sequence thus becomes:

(II)     L-Gly-L-Gly-L-Phe-L-Met-Thr-R$_1$-L-Glu-X-L-Ser-R$_2$-L-Thr-Pro-L-Leu-Val-Thr-R$_3$ wherein X, R$_1$, R$_2$ and R$_3$ are as defined above.

The invention also includes the peptides which consist solely of the sequences according to formula I and II, and functional derivatives thereof.

These.sequences are then :

(III)     H- Thr-R$_1$-L-Glu-X-L-Ser-R$_2$-L-Thr-Pro-L-Leu-Val-Thr-R$_3$-OH

and

(IV)     H-L-Gly-L-Gly-L-Phe-L-Met-Thr-R$_1$-L-Glu-X-L-Ser-R$_2$-L-Thr-Pro-L-Leu-Val-Thr-R-OH

## Description of the preferred embodiments

The peptides of Formula (II), (III) and (IV) are preferred since they are economical to prepare while, in addition, they also give rise to fewer side effects on degradation, since fewer peptide fragments are released.

Of the preferred peptides, peptides wherein X is Nle or Lys-(Ac) are metabolically the most stable.

The best results are obtained in combination with a number of substitutions such as indicated below.

Peptides according to the above formulae, wherein X is Nle, R$_1$ is Ser, R$_2$ is Gln and R$_3$ is Leu.

Peptides according to the above formulae, wherein X is Lys-(Ac), R$_1$ is Ser, R$_2$ is Gln and R$_3$ is Leu.

Peptides according to the above formulae, wherein X is Lys-(Ac), R$_1$ is Pro, R$_2$ is Gln and R$_3$ is Leu.

Peptides according to the above formulae, wherein X is Lys-(Ac), R$_1$ is Pro, R$_2$ is Gln, R$_3$ is Cha and Thr at position 76 is D-Thr.

In the case of the amino acids for which the L or D configuration is not indicated, no preference for either exists. Generally however, the D configuration is more stable.

Starting from the peptides according to the invention, many derived peptides can be prepared without deviating from the spirit of the invention. Many derivatization methods useful in the invention are described in European Patent Application 0,015,036 the contents of which are incorporated by this reference.

As used herein, the following applies:

I. The following abbreviations have been used for the protecting or activating groups employed:

tBu = tertiary butyl

Me = methyl

Z = benzyloxycarbonyl

II. The following abbreviations have been assigned to the solvents or reagents used:

To = toluene

etOH = ethanol

Bu = butanol

HOAc = acetic acid

Am = amyl alcohol

I.A.N. or IAN = iso-amyl nitrite
DMF = dimethylformamide
DCC = dicyclohexylcarbodi-imide
DCU = dicyclohexylurea
TFA = trifluoro-acetic acid
N.E.M. = N-ethylmorpholine
HOBt = N-hydroxybenztriazole
III. The following abbreviations have been used for the amino-acid groups:
Met = methionyl
Phe = phenylalanyl
Pro = prolyl
Ser = seryl
Lys = lysyl
Thr = threonyl
Glu = glutamyl
Gln = glutaminyl
Val = valyl
Leu = leucyl
Ala = alanyl

The other abbreviations are abbreviations for derivatized amino acids which are defined as follows. Cha = cyclohexylalanine; $A_2$hy = 2,6-diamino-hexynoic acid; tyramine = descarboxytyrosine; (HO)Leu = leucic acid; Bua = tertiary butylalanine; Bug = tertiary butylglycine; Lys-(Ac) = Lys-(acetyl); Lys-Z = lysine with a benzyloxycarbonyl group in the $\epsilon$-position; Mag = methallylglycine.

The peptides and peptide derivatives according to the general formula I are prepared in the manner customary for compounds of this type. See, e.g. EP 0,015,036 A1, pages 5-11. The methods most frequently used for the preparation of the compounds in question can be summarized as follows in three alternative methods:

a) a condensation reaction of (1) a compound (amino acid or peptide) with a free carboxyl group and protected other reactive groups with (2) a compound (amino acid or peptide) with a free amino group and protected other reactive groups, in the presence of a condensing agent,

b) a condensation reaction of (1) an amino acid or peptide with an activated carboxyl group and possibly protected other reactive groups with (2) a compound (amino acid or peptide) with a free amino group and possibly protected other reactive groups, and

c) a condensation reaction of (1) an amino acid or peptide with a free carboxyl group and protected other reactive groups with (2) a compound (amino acid, peptide or amine) with an activated amino group and possibly protected other reactive groups, after which the protective groups are removed if desired.

Methods for activating carboxyl groups are known per se and this activation can take place, inter alia, by converting the carboxyl group to an acid halide, an azide anhydride, imidazolide or an activated ester, such as the N-hydroxy-succinimide or p-nitrophenyl ester.

For methods of activation see: Houben-Weyl, Methoden der Organischen Chemie ( Methods of Organic Chemistry ), fourth edition, part XV/2 (Georg Thieme Verlag).

The most customary methods for the above condensation reactions are: the carbodiimide method, the azide method, the mixed anhydride method and the method involving the activated esters, as described in The Peptides , volume I, 1965 (Academic Press) E. Schröder and K. Lübke.

In addition, the so-called "Solid Phase" method of Merrifield, described in J.Am.Chem.Soc. 85 , 2149 (1963), can be used for the preparation of the peptides or peptide derivatives in question. In this case the coupling of the amino acids of the peptide to be prepared preferably starts from the carboxy-terminal side. With this method a solid phase is needed on which reactive groups are located or on which such groups can be applied. This can be, for example, a copolymer of benzene and divinylbenzene with reactive chloromethyl groups, or a polymer phase rendered reactive with hydroxymethylfunction or benzylamine.

A particularly suitable solid phase is, for example, the p-alkoxybenzyl alcohol resin (4-hydroxymethyl, phenoxymethyl-copolystyrene - 1% divinylbenzene resin) described by Wang J.Am.Chem.Soc. 95 (1974), 1328. After synthesis the peptides can be split from this phase under mild conditions.

A resin that contains chloromethyl groups is also a very suitable solid phase. When a solid phase of this type is used, bonding of the first $\alpha$-amino-protected amino acid to the resin takes place via an ester bond.

After synthesis of the desired amino acid sequence, the detaching of the peptide from the resin follows, using, for example, liquid hydrogen fluoride, using trifluoromethanesulphonic acid or using methanesulphonic acid dissolved in trifluoroacetic acid. The peptide can also be removed from the support using a lower alcohol, preferably methanol or ethanol, in which case a lower alkyl ester of the peptide forms directly. Likewise, splitting with the aid of ammonia gives the amide of a peptide according to the invention.

The reactive groups which may not participate in the condensation reaction may be effectively protected by suitable "protective groups", which can very easily be removed again by hydrolysis or reduction. Thus, a carboxyl group can be effectively protected by, for example, esterification with at least a stoichiometrically effective amount of methanol, ethanol, tertiary butanol, benzyl alcohol or p-nitro-benzyl alcohol, or by conversion to an amide, as described, for example, in Houben-Weyl, Methoden der Organischen Chemie (Methods of Organic Chemistry) , 4th edition, part XV/1, page 315 et seq. This latter protective group is, however, very difficult to remove, so preferrably to use this group only in order to protect the carboxyl group of the C-terminal amino acid in the final peptide or the -carboxyl group of glutamic acid.

Groups which can effectively protect an amino group are usually acid groups, for example an acid group derived from an aliphatic, aromatic, araliphatic or heterocyclic carboxylic acid (such as acetic acid, benzoic acid or pyridine-carboxylic acid), or an acid group derived from carbonic acid (such as the ethoxycarbonyl, benzyloxycarbonyl (Boc), t-butyloxycarbonyl or p-methoxy-benzyloxy-carbonyl group), or an acid group derived from a sulphonic acid (such as the benzenesulphonyl or p-toluenesulphonyl group). However, other groups can also be used, such as substituted or unsubstituted aryl or aralkyl groups, for example benzyl and triphenylmethyl or groups such as ortho nitrophenylsulphenyl or 2-benzoyl-1-methylvinyl. (See Houben-Weyl, part XV/1, page 46 et seq.).

It is advisable also to protect the $\epsilon$-amino group of lysine and possibly the hydroxyl group of serine and threonine. However, this latter protection is not always necessary. Customary protective groups in this connection are a tertiary-butyloxy-carbonyl group or a tosyl group for the $\epsilon$-amino group of lysine, and a t-butyl or benzyl group for the hydroxyl group of serine and threonine.

The protective groups can be split off according to various conventional methods, depending on the nature of the particular group, for example with the aid of trifluoroacetic acid, or by mild reduction, for example with hydrogen and a catalyst, such as palladium, or with HBr in glacial acetic acid.

The peptides according to the invention also include the functional derivatives of these peptides which are understood to be:

a) acid addition salts of the peptides;

b) amides of the peptides, specifically C-terminal amides;

c) esters, specifically C-terminal esters;

d) N-acyl derivatives, specifically N-terminal acyl derivatives and in particular N-terminal acetyl derivatives and

e) metal complexes of the peptides.

The salts can be obtained directly from the reaction medium in which the peptides are prepared, or can be prepared subsequently by reaction of the peptide with a base.

The acid addition salts can be obtained directly by isolating the peptide from the desired acid medium, or the peptide obtained can subsequently be converted to an acid addition salt by reaction of the peptide with an acid such as HCl, HBr, phosphoric acid, sulphuric acid, acetic acid, maleic acid, tartaric acid, citric acid, polyglutamic acid, carboxymethylcellulose, etc.

The metal salts, specifically the alkali metal salts, are obtained by reaction of the peptide with the desired metal base, such as NaOH, $Na_2CO_3$, $NaHCO_3$ etc.

N-acyl derivatives are intended to mean specifically the N-terminal acyl derivatives. These derivatives may be prepared by using an amino acid which is already provided with the particular acyl group in the peptide synthesis. The acyl group then also functions as a protective group in the peptide synthesis. In this way the desired acyl derivative is prepared directly. However, it is also possible to introduce the desired acyl group subsequently, by acylating the peptide in the conventional manner. The N-acyl group which is preferably used is the acetyl group.

Esters and amides are likewise preferably prepared by using an amino acid which is already provided with the desired ester or amide group in the peptide synthesis; however, they can also be prepared by subsequently esterifying the peptide obtained in the conventional manner or converting to an amide.

Amides which are preferably used are the unsubstituted amide, the monomethylamide or dimethylamide or the monoethylamide or diethylamide.

The N-alkyl and N,N-dialkyl derivatives, such as the N-monomethyl or N,N-dimethyl derivatives, may be prepared by using the relevant N-monoalkylamino acid or, if desired, N,N-dialkylamino acid in the peptide synthesis.

The metal complexes can be obtained by bringing the peptide into contact with sparingly soluble metal salts, metal hydroxides or metal oxides. The sparingly soluble metal salts used are usually the metal phosphates, metal pyrophosphates and metal polyphosphates.

Metals which can be used with this method are the metals which belong to the B groups of the periodic system, for example cobalt, nickel, copper, iron and preferably zinc, and also metals which belong to the principal groups of the periodic system and are capable of forming complexes, such as magnesium and aluminium. The preparation of these metal complexes takes place in the conventional manner.

Thus, a metal complex can be obtained by adding the peptide and a sparingly soluble metal salt, metal hydroxide or metal oxide to an aqueous medium. The metal complex can also be obtained by adding an alkaline medium to an aqueous solution of the peptide and a soluble metal salt, by which means the insoluble peptide-metal hydroxide complex is formed.

Moreover, the metal complex can be obtained by adding the peptide, a soluble metal salt and a different soluble salt to an aqueous, preferably alkaline, medium, by which means an insoluble peptide-metal salt complex is formed in situ .

The metal complexes can be used directly as suspensions or, for example, can be freeze-dried and subsequently re-suspended.

The invention also includes pharmaceutical preparations which contain peptides according to the invention. The peptides according to the invention can be administered orally, rectally or parenterally. Preferably, the peptides are used in the form of an injection preparation, for which purpose they are dissolved, suspended or emulsified in a suitable fluid. Mixed with suitable auxiliaries or fillers, they can, however, also be brought into a form which is suitable for oral administration, such as pills, tablets and dragees. The peptides in question can also be administered in the form of a suppository or a spray. Administration forms with a protracted action, such as depot injections or implants, can also be chosen.

The peptides are employed in effective amounts, in combination with known excipients, and are preferably used in a dosage of 1 g to 1 mg per kg of body mass per day, depending on the administration form. The preferred dosage for human administration is (subcutaneously) between 1 and 30 mg per day, more particularly between 3 and 10 mg per day.

The preparation of a number of peptides is given in the following examples.

## Example 1.

Preparation of a peptide according to Formula 1 wherein $R_1$ = Pro, X = Lys-(Ac), $R_2$ = Glu and $R_3$ = Leu-OH.

$$\overset{\text{Ac}}{\underset{|}{\text{H-Thr-Pro-Glu-Lys}}}\text{-Ser-Glu-Thr-Pro-Leu-Val-Thr-Leu-OH}$$

$$\underline{1A}\quad \underline{\text{Boc-Thr-Pro-Glu-}\overset{\text{Ac}}{\underset{|}{\text{Lys}}}\text{-N}^2\text{ H}^3}$$

### 1A.1. Z-Lys(Ac)-OMe

3.94 g of Z-Lys(Boc)-OMe are dissolved in 40 ml of methylene chloride. After passing HCl gas through for 1 hour, the solution is evaporated. The residue is dissolved in DMF, after which 1.8 g Ac-ON$_p$ are added and the pH is adjusted to 8.0 with N.E.M. After stirring for 72 hours at room temperature and pH 8.0, the solution is evaporated to dryness and the residue is dissolved in ethyl acetate and washed with 0.1 N HCl and 10% NaCl solution. After drying (Na$_2$SO$_4$), Z-Lys(Ac)-OMe is obtained as an oil in a yield of 100%. Rf in methylene chloride/methanol (9:1) is 0.80 on SiO$_2$.

### 1A.2. Z-Glu(OtBu)-Lys(Ac)-OMe

3.4 g Z-Glu(OtBu)-OH and 2.7 g HOBt are dissolved in 35 ml DMF, after which the mixture is cooled to -10°C. 2.4 g H-Lys(Ac)-OMe.HCl (obtained by hydrogenation of Z-Lys(Ac)-OMe in DMF in the presence of one equivalent HCl using Pd/C as the catalyst) dissolved in 20 ml DMF and equivalent N.E.M. are then added to the cooled mixture. The pH of the mixture is adjusted to 6.5 with N.E.M. and 2.3 g DCC are then added. After stirring for 15 min. at -10°C and for 18 hours at room temperature, DCU is filtered off and the filtrate is evaporated to dryness. The residue is dissolved in 100 ml Ethyl Acetate (EtoAc) and washed successively with 5% KHSO₄ solution, 5% NaHCO₃ solution and with 10% NaCl solution. After drying (Na₂SO₄), the filtrate is evaporated. The residue is recrystallized from ethyl acetate/n-hexane (1:2). Yield 41.3%. M.p. 95-97°C; $[\alpha]_D^{20}$ = -7.7° (c = 1, DMF). Rf in methylene chloride/methanol (9:1) = 0.52 on SiO₂.

### 1A.3. Z-Pro-Glu(OtBu)-Lys(Ac)-OMe

1.12 g Z-Pro-OH and 1.1 g HOBt are dissolved in 15 ml DMF, after which the mixture is cooled to -10°C. 1.74 g H-Glu(OtBu)-Lys(Ac)-OMe.HCl (obtained after catalytic hydrogenation of the corresponding Z-peptide in the presence of one equivalent of HCl) dissolved in 15 ml DMF are then added to the cooled mixture, after which the pH of the mixture is adjusted to 6.7 by adding N.E.M.; 1.01 g DCC is then added. The same operations as indicated under 1A.2 after the addition of DCC are then carried out.
Yield, after stirring with ether, 80.0%. M.p. 111-113°C, $[\alpha]_D^{20}$ = -30.3° (c = 1, DMF). Rf in methylene chloride/methanol (9:1) is 0.61 on SiO₂.

### 1A.4. Boc-Thr-Pro-Glu(OtBu)-Lys(Ac)-OMe

833 mg Boc-Thr-OH and 1.7 g H-Pro-Glu(OtBu)-Lys(Ac)-OMe (obtained after catalytic hydrogenation in DMF) are coupled via the DCC/HOBT method and purified as described under 1A.2. The protected tetrapeptide is obtained as a foam in 68.2% yield. Rf in methylene chloride/methanol (9:1) is 0.28 on SiO₂.

### 1A.5. Boc-Thr-Pro-Glu(OtBu)-Lys(Ac)-N₂H₃

1.5 g Boc-Thr-Pro-Glu(OtBu)-Lys(Ac)-OMe are dissolved in 15 ml methanol, after which 0.88 ml hydrazine.hydrate is added. After stirring for 18 hours at room temperature a further 0.88 ml hydrazine hydrate is added. After stirring for a total of 72 hours, the solution is evaporated. The residue is recrystallized from ethyl acetate/ether (1:1). Yield 96.7%. Rf in methylene chloride/methanol (9:1) is 0.31 on SiO₂.

### 1A.6 Z-Ser-Glu(OtBu)-Thr-Pro-Leu-Val-Thr-Leu-OtBu

H-Leu-Val-Thr-Leu-OtBu [see Recl. Trav. Chim. Pays Bas 98 , 571(1979)] was acylated with one equiv. of Z-Pro-OH using 1.1 equiv. of DCC and 2.0 equiv. of HOBt as described above. Yield of the protected pentapeptide was 80%; Rf in CH₂Cl₂-MeOH-H₂O (70-30-5) is 0.80 on SiO₂. Catalytic reduction in DMF was followed by a DCC/HOBt mediated reaction with Z-Thr-OH as above to give Z-Thr-Pro-Leu-Val-Thr-Leu-OtBu in 93% yield. Rf in Tol-EtOH (4-1) is 0.53 on SiO₂. Z-Glu (OtBu)-OH was coupled to the hexapeptide, after retrieval of the Z group as usual, again using DCC and HOBt; yield was 76,7% (oil) after acid/base extractions. Rf in Tol-EtOH (4-1) is 0.46 on SiO₂. Catalytic hydrogenation followed by acylation of the resulting product with Z-Ser-OH using DCC and HOBt gave the title compound in 48% yield; $[\alpha]_D^{20}$ -40,6 °C (c = 0.5, DMF); m.p. 133-135 °C; Rf in Tol-EtOH is 0.36 on SiO₂.

### 1A.7   H-Thr-Pro-Glu-Lys(Ac)-Ser-Glu-Thr-Pro-Leu-Val-Thr-Leu-OH

0.68 g of Boc-Thr-Pro-Glu(OtBu)-Lys(Ac)-N₂H₃ is dissolved in 10 ml of DMF and 2,2 ml 0.1 N HCl/DMF are added. To the cooled solution for 10 min a solution (-20 °C) 0.15 ml I.A.N. is added; after stirring for 10 min a solution of 0.76 g H-Ser-Glu-(OtBu)-THR-Pro-Leu-Val-Thr-Leu-OtBu (obtained from 1A.6 after catalytic reduction) in 10 ml of DMF is added and the pH adjusted to 7.2 with N.E.M.. After 4 days at 4-8 °C, the protected 12-peptide is isolated by precipitation from ethanol-ethyl acetate-ester (1:1:1) in 70.8%.
0.85 g of the protected peptide is dissolved in 10 ml of TFA-H₂O (91, v/v) and a few drops of amisole, are

7

added. After 1 h at RT ether is added, the formed precipitate filtered off and washed with ether. The crude material is dissolved in tert. BuOH-H₂O (1:1) and an ion-exchange resin in the acetate form added; after stirring for 30 min the resin is removed by filtration and the filtrate hyophilized. Purification by corinter current distribution (solvent system n-BuOH-HOAc H₂O = 4-1-5) gives 230 mg of peptide; Rf in n-BuOH-HOAc H₂O = (4-1-5) gives 230 mg of peptide; Rf in n-BuOH-pyr-HOAc-H₂O (8-3-1-4) is 0.24 on SiO₂.

$$\text{Ac}$$
$$|$$

### 1A.8. H-Thr-Pro-Glu-Lys-Ser-Glu-Thr-Pro-Leu-Val-Thr-Leu-OH

0.68 g Boc-Thr-Pro-Glu(OtBu)-Lys(Ac)-N₂H₃ is dissolved in 10 ml DMF, after which 2.2 ml of 1.0 M HCl/DMF are added. The solution is cooled to -20° C. 0.15 ml I.A.N. is then added and the whole is stirred for 10 min at -20° C. A solution of 0.76 g H-Ser-Glu-Thr-Pro-Leu-Val-Thr-Leu-OtBu in 10 ml DMF is then added. The pH of the reaction mixture is then brought to 7.2 with N.E.M. After standing in the refrigerator for 4 days, the protected dodeca peptide is obtained by precipitation from ethanol/ethyl acetate/ether (1:1:1) in 84.8% yield.

1.06 g of this dodeca peptide are introduced into 12 ml TFA/H₂O(9:1). A few drops of anisole are added to this mixture. The mixture is stirred for 1 hour at room temperature and then poured into ether. The solid is filtered off and then redissolved in tert.BuOH/H₂O (1:1), after which an ion exchanger in the acetate form is added and the mixture is stirred for half an hour. The ion exchanger is filtered off and the filtrate is lyophilized. The substance is purified via counter-current distribution in the system n-BuOH:HOAc:H₂O = 4:1:5. Yield: 330 mg. Rf in n-BuOH:pyridine:HOAc:H₂O (8:3:1:4) is 0.28 on SiO₂.

### Example 2.

Preparation of a peptide according to Formula 1 wherein R₁ = Pro, X = Lys-(Ac), R₂ = Gln, Thr₁₆ = D-Thr and R₃ = Cha-OH.

$$\text{Ac}$$

### H-Thr-Pro-Glu-Lys-Ser-Gln-Thr-Pro-Leu-Val-D-Thr-Cha-OH

For the preparation of Boc-Thr-Pro-Glu(OtBu)-Lys(Ac)-N₂H₃ see Example 1A. 1-5.

#### 2.1 H-Ser-Gln-Thr-Pro-Leu-Val-D-Thr-Cha-OtBu

#### 2.1.1 H-Cha-OtBu.HCl

9.8 g H-Phe-OtBu.HCl are dissolved in 50 ml MeOH. PtO₂ is then added, after which the reaction vessel is shaken for 48 hours with H₂ under elevated pressure (3 bar). PtO₂ is removed and the filtrate is evaporated. H-Cha-OtBu.HCl is obtained as a solid in 94.2% yield. Rf in CH₂Cl₂:MeOH:H₂O (14:6:1) is 0.94 on SiO₂.

#### 2.1.2 H-D-Thr-Cha-OtBu

10.4 g Z-D-Thr-OH and 7.9 g HOBt are dissolved in 100 ml DMF. The whole is cooled to -10° C. 10.28 g H-cha-OtBu.HCl in 180 ml DMF and 1 equivalent T.E.A. are then added to the cooled mixture and subsequently 8.9 g DCC are added. After stirring for 2 hours at 5° C and for 18 hours at room temperature, DCU is filtered off and the filtrate is evaporated to dryness. The residue is dissolved in 125 ml EtOAc and washed with 5% NaHCO₃ solution, 30% NaCl solution, 0.1 M HCl and again 30% NaCl solution. After drying (Na₂SO₄) and filtering off, the filtrate is evaporated to dryness. The residue is recrystallized from ether/n-hexane (1:4). Yield 79.7%, melting point 118-120° C. Rf in To:EtOH(4:1) is 0.66 on SiO₂.

The peptide obtained above is dissolved in MeOH, after which Pd/C (10%) is added and $H_2$ is passed in until no further evolution of $CO_2$ is detected. After filtering off, the filtrate is evaporated. Rf in To:EtOH (8:2) is 0.27 on $SiO_2$.

## 2.1.3 Z-Ser-Glu-Thr-Pro-Leu-Val-OH

33.6 g Z-Ser-Gln-Thr-Pro-OH and 16.2 g H-Leu-Val-OtBu are coupled via the DCC/HOBt method. 33.6 g Z-Ser-Glu-Thr-Pro-OH and 10.5 g HOBt are dissolved in 30 ml DMF, after which the mixture is cooled to -10° C. 16.2 g H-Leu-Val-OtBu.HCl in 30 ml DMF + 1 equivalent N.E.M. are then added to the cooled mixture. The pH of the mixture is adjusted to 6.7 with N.E.M., after which 12.3 g DCC are added. After stirring for 15 minutes at -10° C and for 18 hours at room temperature, DCU is filtered off and the filtrate is evaporated to dryness. The residue is dissolved in 2-BuOH/$CH_2Cl_2$(2:3) and washed successively with 15% NaCl solution, 5% $KHSO_4$ solution, 5% $NaKCO_3$ solution and again with 15% NaCl solution. After drying and filtering off, the filtrate is evaporated to dryness. The substance is obtained after recrystallization from DMF-EtOH-EtOAc, in 48.0% yield. Rf in To:EtOH (8:2) is 0.26 on $SiO_2$.

1.4 g Z-Ser-Gln-Thr-Pro-Leu-Val-OtBu are dissolved in 15 ml 90% TFA, after which a few drops of anisole are added. The free acid is obtained after stirring for 1 1/2 hours and pouring into ether. Yield 84.0%. Rf in $CH_2Cl_2$: $MeOH:H_2O$ (14:6:1) is 0.45 on $SiO_2$.

## 2.1.4 H-Ser-Gln-Thr-Pro-Leu-Val-D-Thr-Cha-OtBu

0.98 g Z-Ser-Glu-Thr-Pro-Leu-Val-OH in 0,26 g HOBt are dissolved in 10 ml DMF, after which the solution is cooled to -10° C. A solution of 0.40 g H-D-Thr-Cha-OtBu in 5 ml DMF is then added followed by 0.27 g DCC . After stirring for 2 hours at 5° C and for 18 hours at room temperature, DCU is filtered off and the filtrate is evaporated. The solid is obtained by precipitation from MeOH/ether/n-hexane (20:60:60), in 67.9% yield; melting point 197-199° C. Rf in To:EtOH (8:2) is 0.24 on $SiO_2$.

The peptide is then dissolved in DMF and one equivalent of HCl, after which Pd/C (10%) is added. $H_2$ is passed in until no further evolution of $CO_2$ is detected. Pd/C is filtered off. Rf in $CH_2Cl_2$:MeOH:Wa (14:6:1) is 0.34 on $SiO_2$.

0.33 g Boc-Thr-Ser-Glu-(OtBu)-Lys(Ac)-$N_2H_3$ and 0.38 g H-Ser-Glu-Thr-Pro-Leu-Val-D-Thr-Cha-OtBu are coupled by the azide method. The protected 12-peptide is isolated by crystallization from ethanol/ethyl acetate/ether (5:5:1) in a quantitative yield.

610 mg of the peptide obtained are introduced into 6 ml TFA/$H_2O$ (9:1), after which a few drops of anisole are added. The mixture is stirred for 1 hour at room temperature and then poured into ether. Further preparation is as is previously described. Yield 400 mg. Rf in n-BuOH:Pyridine:HOAc:$H_2O$ (4:1:1:2) is 0.42 on $SiO_2$.

## Example 3.

Preparation method for a peptide according to Formula 2 wherein $R_1$ = Ser, X = Lys-(Ac), $R_2$ = Gln and $R_3$ = Leu-OH.

```
                    Ac
                    |
H-Thr-Ser-Glu-Lys-Ser-Glu-Thr-Pro-Leu-Val-Thr-Leu-OH
```

```
                    Ac
                    |
H-Thr-Ser-Glu-Lys-Ser-Gln-Thr-Pro-Leu-Val-Thr-Leu-OH
```

3A.1 Z-Thr-Ser-Glu(OtBu)-Lys(Ac)-OMe

4.54 g Z-Thr-Ser-N2H3 and 5.4 g H-Glu(OtBu)-Lys(Ac)-OMe.HCl are coupled via the azide method. After washing, the protected tetrapeptide is obtained by crystallization from ethyl acetate in 84.6% yield. M.p. 148-149°C: $[\alpha]_D^{20}$ = -5.25° (c = 1, DMF). Rf in n-butanol/acetic acid/water (4:1:1) is 0.73 on $SiO_2$.

3A.2 Z-Thr-Ser-Glu(OtBu)-Lys(Ac)-N₂H₃

1.4 g Z-Thr-Ser-Glu(OtBu)-Lys(Ac)-OMe are dissolved in 20 ml MeOH, after which 1.5 ml hydrazine.hydrate are added. After stirring for 18 hours at room temperature, a solid is filtered and, after washing with water, dried. Yield 67.8%. M.p. 193-194°C: $[\alpha]_D^{20}$ = -1.5° (c = 1, DMF). Rf in n-butanol/acetic acid/water (4:1:1) is 0.41 on $SiO_2$.

$$\overset{\displaystyle Ac}{\underset{\displaystyle |}{\phantom{x}}}$$

3A.3   H-Thr-Ser-Glu-Lys-Ser-Glu-Thr-Pro-Leu-Val-Thr-Leu-OH

767 mg Z-Thr-Ser-Glu(OtBu)-Lys(Ac)-N₂H₃ and 0.97 g H-Ser-Gln-Thr-Pro-Leu-Val-Thr-Leu-OtBu are coupled via the azide method and prepared as described in 2.1.4. Yield 746 mg of protected dodeca-peptide. Z-Thr-Ser-Glu(OtBu)-Lys(Ac)-Ser-Glu-Thr-Pro-Leu-Val-Thr-Leu-OtBu is dissolved in DMF, after which Pd/C (10%) is added and $H_2$ is passed in until no further evolution of $CO_2$ is detected. After filtering off the Pd/C, the filtrate is evaporated. The material obtained is dissolved in 10 ml TFA/$H_2O$ (9:1), after which a few drops of anisole are added. The mixture is stirred for 1 1/2 hours at room temperature and then poured into ether.After filtration, the solid is dissolved in tert.BuOH/$H_2O$ (1:1) and an ion exchanger in the acetate form is added, after which the mixture is stirred for half an hour. The ion exchanger is then filtered off and the filtrate is lyophilized. The substance is purified via counter-current distribution in the solvent system n-BuOH:HOAc:$H_2O$ (4:1:5). Yield, after lyophilization, 265 mg. Rf in n-BuOH:pyridine:HOAc:$H_2O$ (8:3:1:4) is 0.29 on $SiO_2$.

Example 4.

Preparation of a peptide according to formula 1 wherein X = Lys-(Ac), $R_1$ = Pro, $R_2$ = Gln and $R_3$ = Leu-OH.

$$Ac$$

H-Thr-Pro-Glu-Lys-Ser-Gln-Thr-Pro-Leu-Val-Thr-Leu-OH

Preparation of: Z-Pro-Glu(OtBu)-Lys(Ac)-OMe

2.5 g Z-Pro-OH and 2.75 g HOBt are dissolved in 25 ml DMF, after which the mixture is cooled to -10°C. 4.82 g H-Glu(OtBu)-Lys(Ac)-OMe.HCL in 40 ml DMF and 1 equivalent M.E.M. are then added to the cooled mixture, the pH of the mixture is adjusted to 6.7 with M.E.M. and 2.3 g DCC are then added. After stirring for 15 min at -10°C and for 18 hours at room temperature DCU is filtered off and the filtrate is evaporated to dryness. The residue is dissolved in 100 ml EtOAc and washed successively with 5% $KHSO_4$ solution, 5% $NaHCO_3$ and with 15% NaCl solution. After drying and filtering off, the filtrate is evaporated to dryness. Recrystallization from EtOAc/petroleum ether (1/2) had no effect, so that the substance was obtained as an oil in 92.7% yield. Rf in To: EtOH (8:2) = 0.26 on $SiO_2$.

Preparation of: Boc-Thr-Pro-Glu(OtBu)-Lys(Ac)-OMe

The tripeptide obtained above is dissolved in DMF + 1 equivalent HCl, after which Pd/C (10%) is added and $H_2$ is passed in until no further evolution of $CO_2$ is detected. After filtering off the Pd/C, the filtrate is used directly.

1.7 g Boc-Thr-OH and 2.1 g HOBt are dissolved in 17 ml DMF, after which the mixture is cooled to -10°C. Subsequently the H-Pro-Glu(OtBu)-Lys(Ac)-OMe.HCl obtained above, in DMF and 1 equivalent N.E.M., is added to the cooled mixture, the pH of the mixture is adjusted to 6.7 with M.E.M. and 1.8 g DCC are then added. The preparation method as described earlier is followed for the further steps. The substance was obtained as an oil in 79.3% yield. Rf in $CH_2Cl_2$:MeOH:Wa (70:30:5) = 0.73 on $SiO_2$

Boc-Thr-Pro-Glu(OtBu)-Lys(Ac)-N$_2$H$_3$

4.6 g Boc-Thr-Pro-Glu(OtBu)-Lys(Boc)-OMe are dissolved in 46 ml ethanol, after which 2.5 ml hydrazine hydrate are added. After stirring for 16 hours, the mixture is evaporated and the residue stirred with EtOAc/ether (1:2). After stirring with $H_2O$, the resulting solid is obtained in 50% yield. Rf in To:EtOH (4:1) = 0.21 on $SiO_2$.

<div align="center">

Ac
|
H-Thr-Pro-Glu-Lys-Ser-Gln-Thr-Pro-Leu-Val-Thr-Leu-OH

</div>

1.1 g of the hydrazide obtained in 5.3 are dissolved in 15 ml DMF, after which 1.50 ml 2.0 M HCl/DMF are added. The solution is cooled to -20°C. 0.23 ml I.A.N. is then added and the mixture is stirred for 10 minutes at -20°C.

1.4 g of the H-Ser-Glu-Thr-Pro-Leu-Val-Thr-Leu-OtBu, obtained via hydrogenation, in 15 ml DMF is added and the reaction mixture is adjusted to pH 7.2 and placed in a refrigerator for 4 days. The mixture is then evaporated. A solid is obtained by precipitation from MeOH/ether (1:5), after which it is purified by column purification ($SiO_2$) in the system n-BuOH: pyridine: HOAc: $H_2O$ = 8:3:1:4 (by volume). Yield 400 mg. Rf in n-Bu:Py:Ac:$H_2O$ (8:3/4:1/4:1) is 0.73 on $SiO_2$.

350 mg of the obtained peptide are introduced into 4 ml 90% TFA. The mixture is stirred for 1 hour at room temperature and then poured into ether. The solid obtained is filtered off, dried, and then dissolved into tert.BuOH/$H_2O$ (1:1), after which an ion-exchanger in acetate form (Lewatit[R]) is added and the mixture is stirred for half an hour. The ion exchanger is filtered off and the filtrate is frozen and freeze-dried. The substance is purified via counter-current distribution in the system n-BuOH:HOAc:$H_2O$ = 4:1:5. Yield 160 mg. Rf in n-BuOH:pyridine:HOAc::$H_2O$ (4:1:1:2) is 0.25 on $SiO_2$.

Example 5.

Preparation of a peptide according to formula 1 wherein X = Nle, $R_1$ = Ser, $R_2$ = Glu and $R_3$ = Leu-OH. H-Thr-Ser-Glu-Nle-Ser-Glu-Thr-Pro-Leu-Val-Thr-Leu-OH

Synthesis of Z-Thr-Ser-Glu(OtBu)-Nle-N$_2$H$_3$

5.1 H-Glu(OtBu)-Nle-OMe

5.15 g Z-Glu(OtBu)-OH and 2.95 g HOBt are dissolved in 30 ml DMF, after which the mixture is cooled to -10°C. 2.6 g H-Nle-OMe.HCl ( J. Med. Chem 16, 1285(1973)) in 20 ml DMF and 1 equivalent M.E.M. are then added to the cooled mixture, the pH of the mixture is adjusted to 6.0 with N.E.M. and 3.3 g DCC are then added. After stirring for 2 hours at 4°C and overnight at room temperature, DCU is filtered off and the filtrate is evaporated to dryness. The residue is dissolved in 100 ml EtOAc and washed successively with 15% NaCl solution, 5% KHSO$_4$ solution, 5% NaHCO$_3$ solution and 15% NaCl solution. After drying and filtering off, the filtrate is evaporated to dryness. The residue is recrystallized from ether/n-hexane. Yield: 81.6%: melting point 48-50°C. Rf in To:EtOH (8:2) is 0.73 on $SiO_2$.

5.2. The peptide obtained is dissolved in DMF, after which Pd/C (10%) and 1 equivalent HCl/DMF are added. $H_2$ is passed in until no further evolution of $CO_2$ is detected. After filtering off, the filtrate is used as such in step 5.3.. Rf in To: EtOH(8:2) is 0.32 on $SiO_2$.

5.3. Z-Thr-Ser-Glu(OtBu)-Nle-N$_2$H$_3$

3.9 g Z-Thr-Ser-N$_2$H$_3$ are dissolved in 30 ml DMF and 7.3 ml 3.0 HCl/DMF. The added clear solution is cooled to -20° C. 1.6 ml I.A.N. are added, after which the mixture is stirred for 20 minutes at -20° C. 4.2 g of the peptide obtained in Example 5.2 in 40 ml DMF are then added and the reaction mixture is adjusted to pH 7.2 and placed in the refrigerator for 4 days. The mixture is then evaporated, the residue dissolved in EtOAc/H$_2$O, and washed with H$_2$O, 5% KHSO$_4$ solution, 5% NaHCO$_3$ and H$_2$O. After drying and filtration, the substance is obtained as an oil in 89.1% yield. Rf in To: EtOH (4:1) is 0.40 on SiO$_2$.

The oil obtained is dissolved in 60 ml MeOH, after which 6.0 ml hydrazine.hydrate are added. After stirring for 18 hours and after adding ether, the solid is filtered off. The solid is recrystallized from MeOH. Yield 64.1%, melting point 207-209° C. Rf in To:EtOH (8:2) is 0.10 on SiO$_2$.

## 5.4.H-Thr-Ser-Glu-Nle-Ser-Glu-Thr-Pro-Leu-Val-Thr-Leu-OH

0.98 g of the hydrazide obtained from Example 5.3 is dissolved in 10 ml DMF and 0.83 ml 3.9 N HCl/DMF. The clear solution is cooled to -20° C. 0.22 ml I.A.N. is then added and the mixture is stirred for 30 minutes at -20° C. 1.05 g H-Ser-Glu-Thr-Pro-Leu-Val-Thr-Leu-OtBu in 10 ml DMF are added and the reaction mixture is adjusted to pH 7.2 and placed in the refrigerator for 4 days. The mixture is poured into water, after which a solid is filtered off. Yield 88.2%, Rf in CH$_2$Cl$_2$:MeOH:H$_2$O (70:30:5) is 0.78 on SiO$_2$.

Z-Thr-Ser-Glu(OtBu)-Nle-Ser-Gln-Thr-Pro-Leu-Val-Thr-Leu-OtBu is dissolved in DMF, after which Pd/C (10%) and 1 equivalent HCl (2N) are added and H$_2$ is passed in until no further evolution of CO$_2$ is detected. After filtering off the Pd/C, the filtrate is evaporated. The hydrogenated peptide is introduced into 11 ml 90% TFA, to which a few drops of anisole are added. The mixture is stirred for 1 1/2 hours at room temperature and then poured into ether. The solid is filtered off and dried. After dissolving in tert.BuOH/H$_2$O (1:1), an ion exchanger in the acetate form is added and the mixture is stirred for half an hour. The ion exchanger is then filtered off and the filtrate is lyophilized. The substance is purified via counter-current distribution in the solvent system Bu:Ac:Wa (4:1:5). The combined fractions are evaporated, the residue is dissolved in 50 ml tert. BuOH/H$_2$O (1:1), and the solution is frozen and freeze-dried. Yield: 240 mg Rf in BuOH:Pyridine:HOAc:H$_2$O (4:1:1:2) is 0.50 on SiO$_2$.

## Example 6.

Preparation of a peptide according to formula 1 wherein X = Lys-(Ac), R$_1$ = Ser, R$_2$ = Gln and R$_3$ = Phe-OH.

### 6.1. H-Thr-Phe-OtBu

2.5 g Z-Thr-OH and 2.7 g HOBt are dissolved in 25 ml DMF, after which the mixture is cooled to -10° C. 2.6 g H-Phe-OtBu-HCl in 20 ml DMF and 1 equivalent N.E.M. are then added to the cooled mixture, the pH of the mixture is adjusted to 6.7 with N.E.M. and 2.3 g DCC are then added. After stirring for 15 min. at -10° C and for 18 hours at room temperature, DCU is filtered off and the filtrate is evaporated to dryness. The residue is dissolved in 100 ml EtOAc and washed with 5% KHSO$_4$ solution, 5% NaHCO$_3$ solution and with 15% NaCl solution. After drying and filtering off, the filtrate is evaporated to dryness. Recrystallization from ether/n-hexane (1:1) gave the protected dipeptide in 91.4% yield. Rf in To:EtOH (4:1) is 0.60 on SiO$_2$.

The peptide obtained is dissolved in DMF, after which Pd/C (10%) is added and H$_2$ is passed in until no further evolution of CO$_2$ is detected. Pd/C is filtered off. Rf in CH$_2$Cl$_2$:MeOH (8:2) is 0.49 on SiO$_2$.

### 6.2. Boc-Thr-Ser-Glu(OtBu)-Lys(Ac)-Ser-Gln-Thr-Pro-Leu-Val-OH

8.19 g Z-Ser-Glu-Thr-Pro-Leu-Val-OH are dissolved in 100 ml MeOH and 1 equivalent HCl, after which Pd/C (10%) is added. H$_2$ is passed in until no further evolution of CO$_2$ is detected. Pd/C is filtered off and the filtrate is evaporated. Rf in AmOH:Pyridine:H$_2$O (5:3:2) is 0.21 on SiO$_2$.

8.43 g Boc-Thr-Ser-Glu(OtBu)-Lys(Ac)-N$_2$H$_3$ and 7.10 g H-Ser-Gln-Thr-Pro-Leu-Val-OH are coupled via the azide method. After precipitation from EtOAc/DMF, the decapeptide is obtained in 44.8% yield. Rf in AmOH:Pyridine:H$_2$O (5:3:2) is 0.70 on SiO$_2$.

6.3. H-Thr-Ser-Glu-Lys(Ac)-Ser-Gln-Thr-Pro-Leu-Val-Thr-Phe-OH

1.3 g Boc-Thr-Ser-Glu(OtBu)-Lys(Ac)-Ser-Gln-Thr-Pro-Leu-Val-OH and 0.36 g H-Thr-Phe-OtBu.HCl are coupled using the DCC/HOBt method already described earlier. The protected 12-peptide is obtained, by precipitation from EtOAc, in 50% yield. Rf in $CH_2Cl_2$:MeOH (8:2) is 0.55 on $SiO_2$.

830 mg of the peptide obtained are dissolved in 8 ml 90% TFA. The mixture is stirred for 1 hour and then poured into ether. Further preparation is analogous to the earlier examples. The yield is 400 mg. Rf in n-BuOH:Pyridine:HOAc:$H_2O$ (4:1:1:2) is 0.32 on $SiO_2$.

Example 7

Preparation method for a peptide according to formula 1 wherein X = Lys-(Ac), $R_1$ = Ser, $R_2$ = Gln, $R_3$ = Leu-OH and $Pro^{13}$ = Δ Pro.

$$\overset{\displaystyle \text{Ac}}{\underset{\displaystyle |}{\text{H-Thr-Ser-Glu-Lys}}}\text{-Ser-Gln-Thr-}\Delta\text{Pro-Leu-Val-Thr-Leu-OH}$$

7.1 H-ΔPro-Leu-Val-Thr-Leu-OtBu

7.1.1 Fmoc-Δ Pro-OH

1.0 g H-Δ Pro-OH (L-3,4-dehydroproline) is dissolved in 50 ml 10% $Na_2CO_3$ solution, after which the solution is cooled in an ice bath. A solution of 2.3 g Fmoc-Cl in 100 ml dioxane is then added dropwise and afterwards the mixture is stirred for three hours at room temperature. After evaporation of the dioxane, the water layer is acidified to pH 2.5 and extracted with ether. After drying over $Na_2SO_4$, hexane is added to the ether solution. Yield 94.6%, melting point 98-100°C.

7.1.2 H-Δ Pro-Leu-Val-Thr-Leu-OtBu

1.0 g Fmoc-Δ Pro-OH and 1.6 g H-Leu-Val-Thr-Leu-OtBu.HCl are coupled in DMF using the DCC/HOBt method already described. The substance is obtained by traturations with EtOH, in a yield of 73.5%; melting point 237-238°C. Rf in To:EtOH (8:2) is 0.53 on $SiO_2$.

7.2. Boc-Thr-Ser-Glu(OtBu)-Lys(Ac)-Ser-Gln-Thr-OH

7.2.1 Boc-Gln-Thr-OBZ1

2.5 g Boc-Gln-OH and 2.5 g H-Thr-OBZ1.HCl are coupled by the DCC/HOBt method already described. The dipeptide is obtained by crystallization from EtOAc/hexane (1:1), in a yield of 100%; melting point 89-91°C. Rf in To:EtOH (8:2) is 0.46 on $SiO_2$.

7.2.2 Boc-Ser-Gln-Thr-OBZ1

a) 4.4 g of the dipeptide obtained under 7.2.1 are dissolved in 44 ml 90% TFA, and the resulting solution is left to stund for 30 minutes at room temperature. The solution is then added dropwise to ether and the resulting oil is evaporated. Rf in $CH_2Cl_2$: MeOH (8:2) is 0.14 on $SiO_2$.

b) 2.0 g Boc-Ser-OH and 4.5 g H-Gln-Thr-OBZ/O-TFA are coupled by the previously described DCC/HOBt method. After the usual washings, the protected tripeptide is isolated by precipitation from EtOAc/ether/hexane (1:1:1), in a yield of 31.7%; melting point 134-136°C. Rf in $CH_2Cl_2$:MeOH (8:2) is 0.77 on $SiO_2$.

EP 0 417 819 A1

## 7.2.3 Boc-Thr-Ser-Glu(OtBu)-Lys(Ac)-Ser-Gln-Thr-OH

a) 1.6 g Boc-Ser-Gln-Thr-OBZl are introduced into 16 ml of TFA and after 30 minutes the solution is added dropwise to ether. Ether is added to the resulting oil and the precipitate formed is filtered off. Yield 96.9%. Rf in $CH_2Cl_2$:MeOH:$H_2O$ (14:6:1) is 0.25 on $SiO_2$.

b) 1.6 g Boc-Thr-Ser-Glu(OtBu)-Lys(Ac)-$N_2H_3$ and 1.6 g H-Ser-Gln-Thr-OBZ1oTFA are coupled to one another using the azide method in the previously described manner. The substance was obtained by crystallization from EtOAc in a 70.4% yield; melting point 166-168° C. Rf in $CH_2Cl_2$: MeOH (9:1) is 0.18 on $SiO_2$.

c) 1.12 g Boc-Thr-Ser-Glu(OtBu)-Lys(Ac)-Ser-Gln-Thr-OBZl are dissolved in DMF. Pd/C (10%) is then added to this solution. After passing in $H_2$ for 18 hours, the Pd/C is filtered off. Rf in $CH_2Cl_2$: MeOH:$H_2O$ (14:6:5) is 0.27 on $SiO_2$.

$$\overset{Ac}{\underset{|}{\phantom{}}}$$

### 7.3. H-Thr-Ser-Glu-Lys-Ser-Gln-Thr-ΔPro-Leu-Val-Thr-Leu-OH

1.01 g Boc-Thr-Ser-Glu(OtBu)-Lys(Ac)-Ser-Gln-Thr-OH and 0.60 g H- Pro-Leu-Val-Thr-Leu-OtBu are coupled by the previously described DCC/HOBt method. After filteration of the DCU, the filtrate is added to $H_2O$. The solid is obtained in a yield of 53.1%. Rf in $CH_2Cl_2$: MeOH (8:2) is 0.71 on $SiO_2$.

850 mg of the protected peptide obtained are introduced into 10 ml 90% TFA. The mixture is stirred for 1 hour at room temperature and then poured into ether. The solid obtained is filtered off, dried and then dissolved in tert.BuOH/$H_2O$ (1:1). After stirring with an ion exchanger, the solution is frozen and freeze-dried. The substance is purified by column chromatography in the system n-BuOH:Pyridine:HOAc:$H_2O$ (8:3:1:4), Yield: 270 mg. Rf in n-BuOH:Pyridine:HOAc:$H_2O$ (4:1:1:2) is 0.37 on $SiO_2$.

## Claims

1. A metabolically stable peptide comprising an amino acid sequence according to the general formula:
Thr-$R_1$-L-Glu-X-L-Ser-$R_2$-L-Thr-Pro-L-Leu-Val-Thr-$R_3$
wherein X is a derivatized lysine selected from the group consisting of Lys-(Ac), Lys(Z), des-Lys, $A_2$hy, Nle, Met, Leu or Lys [(ar)alkyl];
$R_1$ is Ser, Ala or Pro;
$R_2$ is selected from the group consisting of L-Gln, D-Gln, Glu(tyramine) and iodine-containing derivatives thereof; and
$R_3$ is L-Leu, Leu-NHCH$_3$, Met, Phe, Phe(Cl), Phe(I), Cha, Mag, Val, (HO)Leu, Bua or Bug.

2) The peptide according to Claim 1, having the formula; H-Thr-$R_1$-L-Glu-X-L-Ser-$R_2$-L-Thr-Pro-L-Leu-Val-Thr-$R_3$-OH or a functional derivative thereof.

3) The peptide according to Claim 1, having the formula: H-L-Gly-L-Gly-L-Phe-L-Met-Thr-$R_1$-L-Glu-X-L-Ser-$R_2$-L-Thr-Pro-L-Leu-Val-Thr-$R_3$-OH or a functional derivative thereof.

4) The peptide according to Claim 2, wherein X is Lys-(Ac).

5) The peptide according to Claim 2, wherein X is Nle.

6) The peptide according to Claim 4, $R_1$ is Pro, $R_2$ is Gln and $R_3$ is Leu.

7) The peptide according to Claim 4, having the formula: H-Thr-Pro-L-Glu-Lys(Ac)-L-Ser-Gln-L-Thr-Pro-L-Leu-Val-D-Thr-Cha-OH.

8) Peptide according to Claim 5, characterized in that $R_1$ is Ser, $R_2$ is Gln and $R_3$ is Leu.

9) The peptide according to Claim 4, characterized in that $R_1$ is Ser, $R_2$ is Gln and $R_3$ is Leu.

10) A process for preparing metabolically stable, therapeutically useful peptides of the formula (I)
Thr-$R_1$-L-Glu-X-L-Ser-$R_2$-L-Thr-Pro-L-Leu-Val-Thr-$R_3$     (I)
wherein:
X is a derivatized lysine selected from the group consisting of Lys(Ac), Lys(Z), des-Lys, $A_2$hy, Nle, Met, Leu or Lys[(ar)alkyl];
R is Ser, Ala or Pro;
$R_2$ is L-Gln, D-Gln, Glu(tyramine) or iodine containing derivatives thereof, and
$R_3$ is L-Leu, Leu-NHCH$_3$, Met, Phe, Phe(Cl), Phe(I), Cha, Mag, Val, (HO)Leu, Bua or Bug;
or acid addition salts thereof, characterized in that said process comprises:

14

a) coupling two fragments, each fragment comprising one or more amino acids selected from the group Thr, $R_1$, Glu, X, Ser, $R_2$, Pro, Leu, Val and $R_3$, wherein $R_1$, X, $R_2$ and $R_3$ have their previously given meanings, wherein the sequence of amino acids in each of the fragments is the sequence of a part of the peptide of formula (I), and the fragments taken together comprise the complete sequence of the peptide of formula (I), said coupling being by condensation either in a homogenous phase or on a solid phase, and

b) optionally removing any protective groups and/or solid phase from the thus coupled peptide.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 015 036 (AKZO)<br>* Completeley *<br>--- | 1-2 | C 07 K 7/08<br>A 61 K 37/02 |
| X,Y | EP-A-0 004 394 (AKZO)<br>* Completely *<br>--- | 1-3,10 | |
| A | DE-A-3 626 421 (REC. PHARMA VERT.)<br>* Completely *<br>--- | 1 | |
| X | REC. TRAV. CHIM. DES PAYS-BAS, vol. 99, no. 9, 1980, pages 284-286; H.M. GREVEN et al.: "Synthesis of fragments of human beta-lipotropin, betah-LPH. Part IV. The synthesis of shortened peptides related to des-1-tyrosine-gamma-endorphi (beta-LPH-(62-77)), PGS.<br>----- | 1-3,10 | |

TECHNICAL FIELDS
SEARCHED (Int. Cl.5)

C 07 K
A 61 K

The present search report has been drawn up for all claims

| Place of search | Date of completion of the search | Examiner |
|---|---|---|
| THE HAGUE | 24-10-1990 | RAJIC M. |

EPO FORM 1503 03.82 (P0401)